# EUROPÄISCHE PATENTANMELDUNG

(11) **EP 2 787 581 A1**
(43) Veröffentlichungstag der Anmeldung: **08.10.2014**
(21) Anmeldenummer: 13162255.7
(22) Anmeldetag: 04.04.2013
(51) Int. Cl.: H01R 31/02, A61M 1/12

(54) **Implantierbare Kabelverbindungseinrichtung**

(71) Anmelder: Berlin Heart GmbH, 12247 Berlin (DE)
(72) Erfinder: Arndt, Dr., Andreas, 12555 Berlin (DE); Nüsser, Dr., Peter, 14532 Kleinmachnow (DE); Winterwerber, Dr., Kim Peter, 12527 Berlin (DE)
(74) Vertreter: Pfenning, Meinig & Partner GbR

(57) **Zusammenfassung**

Die Erfindung bezieht sich auf eine implantierbare Kabelverbindungseinrichtung mit einem Kabelverbinder (2), der zwei oder mehr Steckverbindungselemente (8, 9, 10) aufweist, mit denen je ein implantierbares Kabel durch eine Steckverbindung verbindbar ist, wobei der Kabelverbinder als steifer Körper aus einem biokompatiblen Material ausgebildet ist.

## Beschreibung

Die Erfindung liegt auf dem Gebiet der Mechanik und Elektrotechnik und ist mit besonderem Vorteil in der Medizintechnik anwendbar. Die Erfindung bezieht sich speziell auf eine Kabelverbindungseinrichtung, die implantierbar ist.

In der Medizintechnik sind unterschiedliche aktive Implantate üblich, die teilweise Körperteile ersetzen oder ergänzen. Sie beinhalten oft Sensoren oder Aktoren und können mechanische oder elektrische Funktionsweisen realisieren. Viele solche Implantate sind mit elektrischen Einrichtungen, beispielsweise Sensoren oder auch Motoren oder Steuerungseinrichtungen, versehen, die, beispielsweise zum Zweck der Energieversorgung oder der Kommunikation, mittels perkutaner Kabel mit dem Bereich außerhalb des Patientenkörpers, in den sie implantiert sind, verbunden werden sollen.

Derartige perkutane Kabel, d. h, Kabel, die in einem Bereich ihres Verlaufs innerhalb des Patientenkörpers und in einem anderen Bereich ihres Verlaufs außerhalb des Patientenkörpers angeordnet sind, stellen grundsätzlich ein Risiko für Infektionen und Entzündungen dar. Es ist daher wünschenswert, dass beim Eintreten von Komplikationen ein entsprechendes Kabel in einfacher Weise entfernt und/oder ausgetauscht werden kann. Zu diesem Zweck ist es grundsätzlich bekannt, innerhalb des Körpers eines Patienten bei einem implantierbaren Kabel eine Steckverbindung vorzusehen.

Aus der internationalen Patentanmeldung WO 2011/156391 A2 ist zu diesem Zweck ein Kabelverbinder bekannt, der als sogenanntes Y-Kabel ausgebildet ist, d. h. mit einem Steckverbinder und einem an diesem unlösbar befestigten Kabel mit mehreren Leitungen, die sich auf zwei getrennte Kabel verzweigen, wobei jedes der getrennten Kabel für sich wieder einen Steckverbinder aufweist. Gemäß der genannten Druckschrift ist ein solcher Kabelverbinder zur elektrischen Verbindung eines perkutanen Kabels mit zwei implantierbaren Pumpen vorgesehen. Die Pumpen dienen zur Förderung von Blut im Blutkreislauf.

Der vorliegenden Erfindung liegt vor dem Hintergrund des Standes der Technik die Aufgabe zugrunde, einen implantierbaren Kabelverbinder zu schaffen, der besonders einfach und stabil aufgebaut ist und bei der Implantation und der Herstellung der Steckverbindungen besonders einfach zu handhaben ist.

Die Aufgabe wird mit den Merkmalen der Erfindung gemäß Patentanspruch 1 gelöst.

Die Erfindung bezieht sich dabei auf eine implantierbare Kabelverbindungseinrichtung mit einem Kabelverbinder, der zwei oder mehr Steckverbindungselemente aufweist, mit denen je ein implantierbares Kabel durch eine Steckverbindung verbindbar ist, wobei der Kabelverbinder als steifer Körper aus einem biokompatiblen Material ausgebildet ist.

Durch die Anordnung mehrerer Steckverbindungselemente an dem Kabelverbinder ist es möglich, diesen vollständig zu implantieren und sowohl ein perkutanes Kabel, das die Verbindung von dem Kabelverbinder zum Körper-äußeren herstellt, als auch ein weiteres, vollständig implantiertes Kabel und gegebenenfalls weitere, insbesondere ebenfalls vollständig implantierte Kabel an den Kabelverbinder anzuschließen. Dadurch, dass dieser als steifer Körper ausgebildet ist, liegen die Eingangs- und Abgangsrichtungen der Steckverbindungselemente zuverlässig fest, und zum Herstellen und Lösen der Steckverbindungen ist es lediglich notwendig, den Kabelverbinder kurzfristig oder dauerhaft zu fixieren. Der Kabelverbinder kann sich nicht im Gebrauch verformen, und hierdurch ist auch verhindert oder erschwert, dass die eingesteckten Kabel durch eine Verformung des Kabelverbinders in eine ungewollte Position gebracht werden. Zudem ist der Kabelverbinders als steifer Körper sehr kompakt herstellbar und leicht in einem Patientenkörper unterzubringen.

Eine besonders vorteilhafte Ausgestaltung der Erfindung sieht vor, dass mindestens zwei, insbesondere drei oder mehr Steckverbindungselemente auf derselben Seite des Kabelverbinders angeordnet sind. Durch diese Geometrie lassen sich mehrere Kabel an die Steckverbindungselemente des Kabelverbinders anstecken, wie es anatomisch günstig ist, insbesondere ohne diesen drehen zu müssen. Dies ist insbesondere dann wichtig, wenn Kabel nach dem Einsetzen des Kabelverbinders in den Patientenkörper gesteckt werden müssen, beispielsweise auch bei der Auswechslung von defekten Kabeln.

Eine weitere vorteilhafte Ausgestaltung der Erfindung sieht vor, dass alle Steckverbindungselemente auf derselben Seite des Kabelverbinders angeordnet sind. Bei dieser Ausgestaltung des Kabelverbinders kann dieser so implantiert werden, dass die Steckverbindungselemente jeweils auf der Seite des Kabelverbinders liegen, die durch einen minimalen Eingriff durch eine minimale Öffnung des Patientenkörpers leicht von außen zu erreichen sind. Der Kabelverbinder selbst muss dann zum Auswechseln der Steckverbindungen nicht dem Patientenkörper entnommen werden. Die Steckverbindungselemente können vorteilhaft an den kleinsten oder schmalsten der Seitenflächen des Kabelverbinders angeordnet sein.

Die Erfindung kann außerdem vorteilhaft dadurch ausgestaltet werden, dass die Steckachsen der Steckverbindungselemente, die gemeinsam auf einer Seite des Kabelverbinders angeordnet sind, parallel zueinander ausgerichtet sind. Auch durch eine parallele, insbesondere steife Anordnung der Steckachsen der Steckverbindungselemente an dem Kabelverbinder lässt sich die Handhabung der Steckverbindungen vereinfachen. Unter den Steckachsen der Steckverbindungselemente wird jeweils die Richtung verstanden, in der ein Kabel mit dem jeweils komplementären Steckverbindungselement auf das an dem Kabelverbinder angeordnete Steckverbindungselement aufgesteckt wird. Bei gängigen hermaphrodisischen Steckverbindungen ist die Steckachse parallel zur Längsachse der Steckerstifte bzw. der entsprechenden Buchsen.

Eine parallele Ausrichtung der Steckachsen der Steckverbindungselemente führt auch dazu, dass diese mit minimalem Abstand zueinander ausgeführt werden können und dass die entsprechenden komplementären Steckverbindungselemente von Kabeln in gleicher Weise mit minimalem Abstand zueinander außerhalb des Kabelverbinders angeordnet werden können. Der insgesamt verwendete und benötigte Bauraum der Kabelverbindungseinrichtung wird somit minimiert.

In besonders vorteilhafter Form wird die Erfindung außerdem dadurch verwirklicht, dass der Kabelverbinder eine als ebene Fläche ausgebildete Seite aufweist, an der die Steckverbindungselemente angeordnet sind, und dass alle übrigen Begrenzungsflächen des Kabelverbinders konvex gerundet sind. Ein derartiger, weitgehend an seinen Außenseiten abgerundet ausgeführter Kabelverbinder lässt sich ohne weiteres, insbesondere ohne die Gefahr von besonderen Gewebebelastungen in den abgerundeten Bereichen des Kabelverbinders, in einen Patientenkörper implantieren. Auch bei einer Bewegung des Gewebes, in das der Kabelverbinder eingebettet ist, sind keine Verletzungen zu befürchten.

Es kann außerdem vorteilhaft vorgesehen sein, dass der Kabelverbinder im Wesentlichen halbkugelförmig abgerundet ausgebildet ist. Außer einer Halbkugel kann der Kabelverbinder auch die Form eines halbierten ellipsoiden oder eines anderen abgerundeten Körpers annehmen.

Besonders vorteilhaft ist auch eine flache Form des Kabelverbinders, bei der die Länge und Breite ein Mehrfaches der Höhe betragen, wobei die Ecken und Kanten abgerundet sind. Dabei kann die Grundform die eines Quaders sein.

Eine weitere vorteilhafte Ausgestaltung der Erfindung sieht vor, dass der Kabelverbinder als Gehäuse mit wenigstens einem Innenraum ausgebildet ist. Ein als kompakter Körper hergestellter Kabelverbinder kann einerseits durch die günstige interne Führung von Leitungen auf kleinem Raum die verschiedenen Steckverbindungselemente miteinander verbinden und zudem noch einen Innenraum aufweisen, in dem vorteilhaft beispielsweise ein elektrischer Energiespeicher/Akkumulator oder ein elektrisch aktives Element, beispielsweise ein elektrischer Schaltkreis, insbesondere eine Einrichtung zur drahtlosen Kommunikation, angeordnet sein kann. Beispielsweise kann in dem Kabelverbinder auch ein Transponder angeordnet sein, der eine Identifikation erlaubt, ohne dass eine Energieversorgung notwendig ist (radio-tag). Der Innenraum kann mit einem Kleber, einer Vergussmasse oder einem Fluid gefüllt sein, es kann jedoch auch ein Hohlraum frei bleiben.

In dem Innenraum kann beispielsweise auch eine Steuereinrichtung für einen oder mehrere Blutpumpenmotoren angeordnet sein. Dazu kann dort ein Mikrocontroller oder eine andere programmierbare Einrichtung vorgesehen sein.

Um eine besonders gewebeverträgliche Ausführungsform der Erfindung zu schaffen, kann zudem vorgesehen sein, dass der Kabelverbinder an seinen Außenseiten ausschließlich aus Metall und/oder Glas und/oder Keramik besteht. Diese Materialien sind in höchstem Maße biokompatibel, d. h. gewebeverträglich, und langzeitstabil, so dass ein aus einem solchen Material bestehender Kabelverbinder langzeitimplantierbar ist. Die Außenflächen sind vorteilhaft glatt gestaltet.

Als Metall kommt in diesem Zusammenhang besonders vorteilhaft Titan in Frage. Das Gehäuse des Kabelverbinders kann dabei auch aus mehreren Teilen bestehen, die zur Außenseite des Kabelverbinders gasdicht zusammengefügt, insbesondere miteinander verschweißt, verklebt oder vergossen sind. Beispielsweise kann ein Metallelement des Kabelverbinders mit einem Glasteil verschmolzen sein. Der Glasteil kann seinerseits wieder mit einer Keramik gasdicht verschmolzen sein. Ein Metall kann auch mit einer Keramik oder einem Glas mittels einer Metalllötung gasdicht verbunden werden. All diesen Verbindungstechniken ist gemein, dass sie eine zuverlässige Fixierung bei gleichzeitiger gasdichter Abdichtung und chemischer sowie physikalischer Festigkeit verbinden.

Ein Kabelverbinder kann beispielsweise, wenn er zusammen mit einer oder mehreren herzunterstützenden Blutpumpen verwendet wird, im Zwischenraum zwischen dem dann üblicherweise etwas verkleinerten Herzmuskel und dem Herzbeutel untergebracht werden. Es ist auch eine subkutane Anordnung des Kabelverbinders möglich, und es können an diesem beispielsweise Ösen zum Vernähen am Körpergewebe vorgesehen sein.

Die Erfindung bezieht sich zudem auf eine Kabelverbindungseinrichtung mit einem Kabelverbinder, einem in diesen eingesteckten ersten perkutanen Kabel und wenigstens einem in den Kabelverbinder eingesteckten zweiten Kabel sowie einem mit dem zweiten Kabel verbundenen Implantat, insbesondere einer Blutpumpe. Zudem kann optional noch ein mit dem Kabelverbinder durch eine Steckverbindung verbundenes drittes Kabel sowie ein mit diesem dritten Kabel verbundenes weiteres Implantat, insbesondere ebenfalls eine implantierte Blutpumpe, vorgesehen sein.

Die Kabelverbindungseinrichtung kann also potenziell nicht nur den Kabelverbinder mit Steckverbindungseinrichtungen umfassen, sondern auch die entsprechenden in diesen eingesteckten Kabel und mit diesen verbundene Blutpumpen oder alternative Implantate. Auch ein eingestecktes perkutanes Kabel, das mit dem Kabelverbinder verbunden ist, kann zu der Kabelverbindungseinrichtung gehören.

Die Kabelverbindungseinrichtung kann somit insgesamt ein System aus mehreren Implantaten, insbesondere Pumpen, umfassen, die mittels Kabelverbindungen über einen Kabelverbinder mit einem perkutanen Kabel verbunden sind, das außerhalb des Patientenkörpers an einem Kabelanschluß einfach zugänglich ist.

Der Kabelverbinder, insbesondere sein Gehäuse, kann zudem wenigstens ein, insbesondere zwei Koppelelemente, beispielsweise Schraubengewinde, Bajonettaufnahmen oder Ähnliches für ein Fixierelement aufweisen, mittels dessen der Verbinder gut manipulierbar ist, besonders auch bei einem Kabelsteckvorgang.

Eine vorteilhafte Ausgestaltung der Erfindung sieht zudem vor, dass an der Außenseite des Kabelverbinders wenigstens zwei Steckverbindungselemente nebeneinander vorgesehen sind und dass zudem eine Verriegelungseinrichtung vorgesehen ist, die, solange jeweils eine der Steckverbindungen gelöst ist, das Lösen der anderen Steckverbindung blockiert, wobei hierzu insbesondere ein Verriegelungselement vorgesehen ist, das zwei Abdeckelemente zur wenigstens teilweisen Abdeckung je einer Steckverbindung aufweist, wobei das Verriegelungselement zwischen zwei Endpositionen bewegbar ist und in jeder der Endpositionen jeweils eine Steckverbindung blockiert ist.

Eine solche Verriegelungseinrichtung verhindert, dass unbeabsichtigt zwei Steckverbindungen an dem Kabelverbinder gleichzeitig gelöst werden, was dazu führen kann, dass beim Wiedereinstecken Kabel vertauscht werden. Dies ist insbesondere dann ungewollt und riskant, wenn zwei Kabelverbindungen zu zwei Blutpumpen gelöst werden, die in verschiedenen Teilen eines Patientenherzens oder an verschiedenen Stellen bezüglich des Herzens in dessen Peripherie oder allgemein im Blutkreislauf angeordnet sind, deren Vertauschung schädlich sein kann. Zusätzlich oder alternativ zu dieser Maßnahme kann auch eine mechanische Kodierung der Steckverbinder zu deren Unterscheidung vorgesehen sein.

Ein vorteilhaftes Verfahren zum Implantieren einer Kabelverbindungseinrichtung der erfindungsgemäßen Art kann außerdem vorsehen, dass der Kabelverbinder im Patientenkörper angeordnet und danach wenigstens ein, insbesondere zwei oder drei implantierbare Kabel mittels Steckverbindungen mit dem Kabelverbinder verbunden werden.

Weitere implantierbare Elemente, wie Blutpumpen, die mit dem Kabelverbinder verbunden werden sollen, können vorteilhaft schon vor der Implantation des Kabelverbinders oder auch nach der Implantation des Kabelverbinders implantiert werden, jedenfalls müssen sie nicht gleichzeitig mit dem Kabelverbinder eingebracht werden.

Die Erfindung bezieht sich auch auf ein Verfahren zum Austauschen mehrerer mit einem erfindungsgemäßen Kabelverbinder verbundener implantierbarer Kabel, bei dem zuerst die Steckverbindung eines ersten Kabels gelöst und ein neues erstes Kabel mit dem Kabelverbinder mittels der Steckverbindung verbunden wird und darauf die Steckverbindung eines zweiten Kabels gelöst und eine Steckverbindung des Kabelverbinders zu einem neuen zweiten Kabel hergestellt wird, wobei insbesondere das erste und das zweite Kabel mit je einer Blutpumpe verbunden sind. Durch ein derartiges Vorgehen wird die Vertauschung zweier Kabel und/oder Steckverbindungen verhindert.

Außerdem bezieht sich die Verbindung auch auf ein Verfahren zum Austauschen eines mit einem erfindungsgemäßen Kabelverbinder verbundenen implantierten Kabels, bei dem zuerst mit einem Koppelelement des Kabelverbinders ein Haltewerkzeug verbunden, darauf die Steckverbindung des Kabels gelöst und ein neues Kabel mittels einer Steckverbindung mit dem Kabelverbinder verbunden wird. Auf diese Weise kann der Kabelverbinder als steifer Körper problemlos von außen festgehalten werden, während Steckverbindungen gelöst oder gesteckt werden. Der Kabelverbinder kann dabei implantiert bleiben, und er kommt nur minimal mit körperfremden Instrumenten in Kontakt.

Ein Koppelelement kann grundsätzlich eine mechanische Passung aufweisen, wie ein Schraubengewinde oder einen Bajonettverschluss, oder es kann beispielsweise durch einen an dem Kabelverbinder und/oder einem Haltewerkzeug befestigten Magneten auch magnetisch ausgebildet sein.

Eine vorteilhafte Verwendung der Kabelverbindungseinrichtung sieht vor, dass die Kabelverbindungseinrichtung mit zwei Herzunterstützungspumpen verbunden und gemeinsam mit diesen unmittelbar an einem Herzen angeordnet, insbesondere an diesem oder am Herzbeutel fixiert ist.

Ein vorteilhaftes Verfahren zum Implantieren einer Kabelverbindungseinrichtung sieht vor, dass der Kabelverbinders mit zwei Herzunterstützungspumpen elektrisch verbunden und zeitlich vorher oder danach am Herzbeutel eines Patientenherzens fixiert wird.

Im Folgenden wird die Erfindung anhand eines Ausführungsbeispiels in einer Zeichnung gezeigt und nachfolgend beschrieben. Dabei zeigt
- Fig. 1: in einer Übersichtsdarstellung eine Kabelverbindungseinrichtung mit zwei Blutpumpen und einem perkutanen Kabel im implantierten Zustand,
- Fig. 2: einen Kabelverbinder im Querschnitt mit drei Steckverbindungselementen,
- Fig. 3: einen Kabelverbinder im Querschnitt mit zwei Steckverbindungselementen und einer ebenen Frontfläche,
- Fig. 4: einen Kabelverbinder mit einer gekrümmten Frontfläche und zwei Steckverbindungselementen,
- Fig. 5: einen Kabelverbinder mit detaillierterer Darstellung der internen Leiterführung,
- Fig. 6: eine Verriegelungseinrichtung für zwei Steckverbindungen an einem Kabelverbinder, sowie
- Fig.7 bis Fig. 12: verschiedene Szenarien zur Platzierung von jeweils zwei Herzunterstützungspumpen für die Herzventrikel und einen Kabelverbinder.

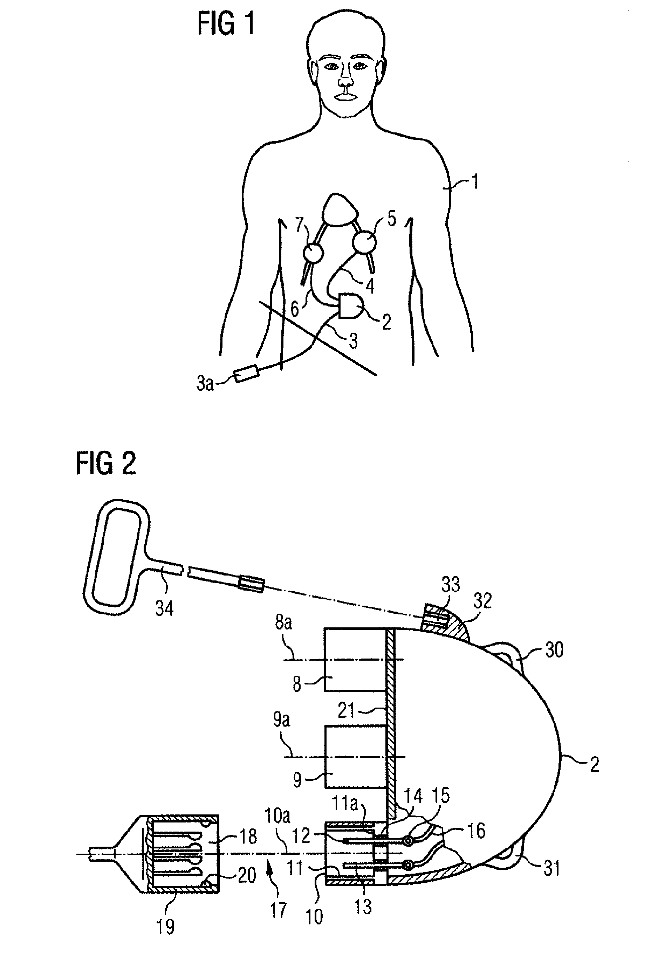

**Figur 1** zeigt schematisch in einer Frontansicht einen Patientenkörper 1, in den ein Kabelverbinders 2 einer Kabelverbindungseinrichtung implantiert ist. Der Kabelverbinder 2 ist mittels eines ersten implantierbaren Kabels 3, das als perkutanes Kabel ausgeführt ist, mit einem ersten Steckanschluss 3a außerhalb des Patientenkörpers verbunden.

Der Begriff "perkutanes Kabel" bezeichnet üblicherweise ein Kabel, das die Haut oder einen anderen Teil der Begrenzungsfläche zwischen dem Patientenkörper und der Außenwelt durchdringt. Im Zusammenhang mit der vorliegenden Erfindung kann ein perkutanes Kabel jedoch auch ein implantierbares Kabel bezeichnen, das vollständig innerhalb des Patientenkörpers verläuft und an einer Anschlusseinrichtung endet, mit der ein außerhalb des Patientenkörpers weiterführendes Kabel beispielsweise induktiv durch die Haut koppelbar ist. Es kann auch vorgesehen sein, dass nur ein Steckanschluss 3a des perkutanen Kabels in die Haut eingelassen ist und diese durchdringt.

An den Kabelverbinder 2 ist außer dem perkutanen Kabel 3 ein zweites implantierbares Kabel 4 angeschlossen, das den Kabelverbinder 2 mit einer ersten Blutpumpe 5 verbindet. Ein drittes implantiertes Kabel 6 ist mit dem Kabelverbinder 2 einerseits und einer zweiten Blutpumpe 7 andererseits verbunden. Die implantierten Kabel 3, 4, 6 sind mit dem Kabelverbinder 2 jeweils über Steckverbindungen elektrisch leitend verbunden. Vorteilhaft sind die implantierten Kabel an ihren dem Kabelverbinder 2 abgewandten Enden mittels je eines weiteren Steckverbinders 3a jeweils mit einem weiteren Element, beispielsweise mit den Blutpumpen 5, 7 verbunden. Die implantierten Kabel 4, 6 können jedoch mit den jeweiligen Blutpumpen 5, 7 auch dauerhaft und unlösbar verbunden sein.

Da mittels des Kabelverbinders 2 mehrere implantierte Elemente, im dargestellten Beispiel die beiden Blutpumpen 5, 7, über ein einziges, perkutanes Kabel 3 von außerhalb des Patientenkörpers ansteuerbar sind, wird der für die Durchführung eines Kabels benötigte Bereich der Haut, der geöffnet wird und dauerhaft von einem Fremdkörper durchsetzt ist, minimiert. Eine besonders vorteilhafte Ausgestaltung ergibt sich dadurch, dass das perkutane Kabel 3 im Querschnitt kreisrund ausgeführt ist, um die Berührungsfläche zwischen dem Kabel und der Haut des Patientenkörpers insgesamt und speziell an der Durchtrittsstelle durch die Haut zu minimieren.

**Figur 2** zeigt schematisch und teilweise im Schnitt einen Kabelverbinder 2 in detaillierterer Darstellung, wobei der Kabelverbinder 2 drei Steckverbindungselemente 8, 9, 10 aufweist. Die einzelnen Steckverbindungselemente am Kabelverbinder 2 weisen jeweils einen hohlen Metallzylinder 11 auf, der als Steckerhülse am Kabelverbinder 2 befestigt ist und der einen Zylinderboden 11a aufweist, der mit dem Kabelverbinder 2 fluiddicht verbunden ist. Der Zylinderboden 11a weist Ausnehmungen auf, durch die Steckerstifte 12, 13 elektrisch isoliert und fluiddicht dadurch durchgeführt sind, dass sie in dem Zylinderboden 11a mittels einer Glasfüllung 14 fluiddicht vergossen sind. Im Inneren des Kabelverbinders 2, wo dieser einen Hohlraum aufweist, sind die Steckerstifte 12, 13 jeweils über Lötstellen 15 mit einer Leitung, beispielsweise einer Kupferlitze 16, verbunden, die ihrerseits den Kontakt der Steckerstifte 12, 13 mit anderen Steckerstiften der übrigen Steckverbindungselemente 8, 9 oder elektronischen Elementen im Innenraum des Kabelverbinders 2 oder einem Energiespeicher im Innenraum des Kabelverbinders 2 herstellt.

Der auf den Metallzylinder 11 am Kabelverbinder 2 aufgesteckte Stecker 17 weist Steckerhülsen 18 auf, die auf die Steckerstifte 12, 13 aufsteckbar sind, um einen galvanischen Kontakt zu diesen herzustellen. Die Steckerhülsen 18 sind ihrerseits mit Leitern verbunden, die innerhalb des Steckers 17 zu dem angeschlossenen implantierten Kabel geführt sind. Es ist ein Steckergehäuse 19 vorgesehen, das eine Form aufweist, die dem Zylinder 11 entspricht und die auf den Zylinder 11 aufsteckbar ist. Dabei ist in dem Steckergehäuse 19 in einer innen umlaufenden Nut ein O-Ring 20 aus einem Elastomer vorgesehen, der an dem Metallzylinder 11 außen fluiddicht dichtet.

Zudem kann eine Sicherung vorgesehen sein, die den Stecker 17 an dem Kabelverbinder 2 festhält, beispielsweise durch eine Überwurfmutter, die an dem Kabelverbinder 2 verschraubbar ist, oder durch eine Bajonettverbindung zwischen dem Stecker 17 und dem Metallzylinder 11.

**Figur 2** zeigt, dass der Kabelverbinder 2 eine ebene Frontfläche 21 aufweist, an der die Steckverbinderelemente 8, 9, 10 derart fixiert sind, dass ihre Steckachsen 8a, 9a, 10a parallel zueinander verlaufen.

Der Gehäuseteil des Kabelverbinders 2, der sich an die ebene Frontplatte anschließt, welche die ebene Frontfläche 21 bildet, ist beispielsweise als Kugelkalotte oder als Teil eines Rotationsellipsoids ausgebildet und besteht beispielsweise aus Metall, insbesondere Titan, oder einer Keramik. Die Verbindungen zwischen dem Metall und der Keramik können durch Aufschmelzen des Metalls oder durch Zwischenlagern eines Glaslots oder Metalllots fluiddicht ausgeführt werden. Es ist auch eine fluiddichte Verpressung der Elemente durch Druckanwendung denkbar.

In Fig. 2 sind auch beispielhaft Koppelelemente, nämlich Halteösen 30, 31, dargestellt, sowie ein Gehäuseansatz 32, in dem sich eine Gewindebohrung 33 befindet, in die zur Manipulation ein Haltewerkzeug 34 eingeschraubt werden kann.

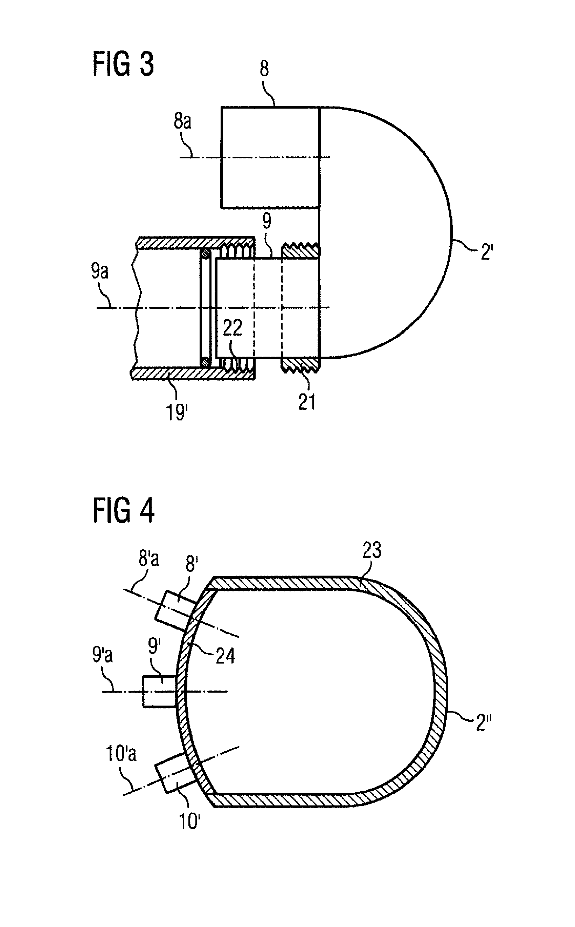

**Figur 3** zeigt einen Kabelverbinder 2' mit nur zwei Steckverbindungselementen 8, 9 an einer ebenen Frontfläche, wobei die Steckverbindungselemente 8, 9 mit ihren jeweiligen Steckachsen 8a, 9a parallel zueinander ausgerichtet sind. An dem Steckverbindungselement 9 ist im Sockelbereich nahe der Frontfläche des Kabelverbinders 2' ein Außengewinde 21 am Metallzylinder dargestellt, das zur Befestigung der Steckverbindung mit einem Innengewinde 22 eines Steckergehäuses 19' zusammenwirkt.

**Figur 4** zeigt im Vergleich einen Kabelverbinder 2" mit einem abgerundeten Gehäuseteil 23 und einer Frontplatte 24, die ebenfalls abgerundet ist, dabei jedoch eine schwächere Rundung aufweist als der Gehäuseteil 23. An der Frontplatte 24 des Kabelverbinders 2" sind drei Steckverbindungselemente 8', 9', 10' befestigt. Diese sind aufgrund der Krümmung der Frontplatte 24 mit ihren Steckachsen 8'a, 9'a, 10'a nicht parallel zueinander, sondern in einem Winkel zwischen 10° und 30° zueinander angeordnet. Dennoch sind alle Steckelemente von einer Seite des Kabelverbinders 2" her steckbar und auch wieder lösbar.

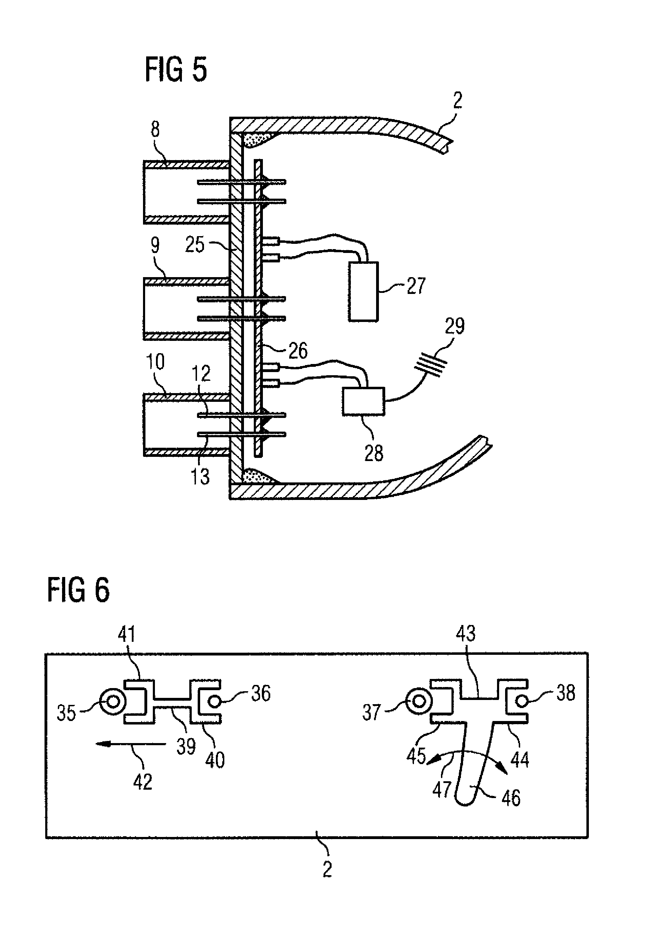

In **Figur 5** ist ein Kabelverbinder mit Steckverbindungselementen 8, 9, 10 dargestellt, wobei dort Steckerstifte 12, 13 die Frontplatte 25 des Kabelverbinders gasdicht, beispielsweise abgedichtet über geschmolzenes Glas, durchsetzen. Die Steckerstifte 12, 13 sind im Innenraum des Gehäuses des Kabelverbinders 2 fortgesetzt und durchsetzen dort eine Leiterplatte 26. Die Steckerstifte 12, 13 sind mit der Leiterplatte 26 und verschiedenen auf dieser befindlichen Leiterbahnen verlötet. Auf der Leiterplatte 26 können zudem elektrische oder elektronische Elemente sowie eine Leiterbahnenführung vorgesehen sein, die wunschgemäß verschiedene Steckerstifte des Kabelverbinders 2 miteinander oder mit einem elektronischen Bauelement verbindet, das sich im Innenraum des Kabelverbinders 2 befindet. Es kann dort auch ein elektrischer Energiespeicher in Form eines Akkumulators 27 vorgesehen und mit Leiterbahnen der Leiterplatte 26 verbunden sein.

Mit der Leiterplatte 26 kann über Steckerstifte auch eine elektronische Einrichtung 28 verbunden sein, die der drahtlosen Kommunikation zwischen Implantaten, die an den Kabelverbinder angeschlossen sind, und einer Einrichtung außerhalb des Patientenkörpers dient, Zu diesem Zweck ist die elektronische Einrichtung 28 mit einer Antenne 29 innerhalb des Kabelverbinders bzw. eines Hohlraums im Kabelverbinder 2 versehen.

Die erfindungsgemäße Kabelverbindungseinrichtung mit einem Kabelverbinder und entsprechenden implantierten Kabeln sowie gegebenenfalls an diese angeschlossenen implantierten Blutpumpen oder anderen Elementen dient dem sicheren Betrieb der Implantate, wobei beim Auftreten von Fehlern in der Anschlusstechnik in einfacher Weise Kabel ersetzt werden können, ohne dass implantierte, an die Kabel angeschlossene Elemente notwendigerweise entfernt bzw. ersetzt werden müssen.

Figur 6 zeigt in einer Frontalansicht einen Kabelverbinder 2 mit Steckverbindungselementen 35, 36, 37, 38 in Form von Steckerstiften, die jeweils von Steckhülsen umgeben sind und auf die entsprechende weibliche Kabelstecker aufsteckbar sind. Die Kabelstecker können beispielsweise im gesteckten Zustand gegen unbeabsichtigtes Abziehen durch eine Überwurfmutter oder einen Bajonettverschluss gesichert sein.

In der Figur ist für die Steckverbindungselemente 35, 36 ein Verriegelungselement 39 dargestellt, das zwei Abdeckelemente 40, 41 aufweist, von denen das Abdeckelement 40 das Steckverbindungselement 36 in der gezeigten Stellung abdeckt und damit blockiert, indem es ein Anfassen/Drehen eines aufgesteckten Kabelsteckers verhindert. Wird das Verriegelungselement 39 nach links in Richtung des Pfeils 42 verschoben, so deckt das Abdeckelement 41 das Steckverbindungselement 35 ab. Somit ist, wenn das Verriegelungselement 39 zwischen den Endpositionen verschoben wird, jeweils eines der Steckverbindungselemente 35, 36 blockiert, so dass zumindest nicht unbeabsichtigt beide Steckverbindungen gelöst werden. Das Verriegelungselement kann beispielsweise in einer Führung auf dem Kabelverbinder 2 verschiebbar geführt sein.

In Fig. 6 ist alternativ im rechten Teil des Kabelverbinders 2 ein Verriegelungselement 43 gezeigt, das zwei Abdeckelemente 44, 45 für die Steckverbindungselemente 37, 38 aufweist, die wechselweise beim Schwenken um die Schwenkachse 46 in Richtung des Pfeils 47 abgedeckt und blockiert werden. Mittels einer an sich bekannten federnden Kniehebelmechanik oder anderer bekannter Mittel können die Verriegelungselemente 39, 43 auch bistabil gehalten sein, so dass sie nur jeweils in ihren Endpositionen unter Abdeckung jeweils eines Steckverbindungselements stabilisiert sind.

Alternativ oder zusätzlich zu einer Verriegelungseinrichtung der oben beschriebenen Art kann auch ein Bussystem vorgesehen sein, das die Adressierung eines bestimmten, über ein Kabel an den Kabelverbinder angeschlossenen Implantats, insbesondere einer Blutpumpe, unabhängig davon ermöglicht, in welches Steckverbindungselement am Kabelverbinder das Kabel eingesteckt ist.

Zusätzlich zu oder anstelle des Kabels 3, das perkutan ausgebildet und mit dem Kabelverbinder 2 verbunden ist, kann noch ein weiteres implantiertes Kabel mit dem Kabelverbinders 2 mittels einer Steckverbindung verbunden sein, das andererseits mit einem Energieversorgungs- oder Kommunikationsmodul verbunden ist, welches direkt unter der Haut eines Patienten angeordnet ist und perkutan über Felder und/oder Wellen eine Energie- oder Informationsübertragung erlaubt. Grundsätzlich kann jedes an dem Kabelverbinder angeschlossene Kabel an seinem dem Kabelverbinder abgewandten Ende mit einem oder mehreren parallelen weiterführenden Kabeln im Sinne einer Kaskadierung verbunden sein.

Die Kabelverbindungseinrichtung, die, wie in Fig. 1 dargestellt, wenigstens ein perkutanes Kabel 3 und ein vollständig implantierbares Kabel 4 aufweist, ist vorteilhaft mit Kabeln der folgenden Kabellängen versehen, insbesondere wenn ein oder zwei implantierte Kabel mit Blutpumpen verbunden sind:
Perkutanes Kabel: 100 bis 160 cm Länge; erstes implantierbares Verbindungskabel zu einem Implantat: 20 bis 40 cm Länge; sowie zweites implantierbares Verbindungskabel zu einem weiteren Implantat: 20 bis 40 cm.

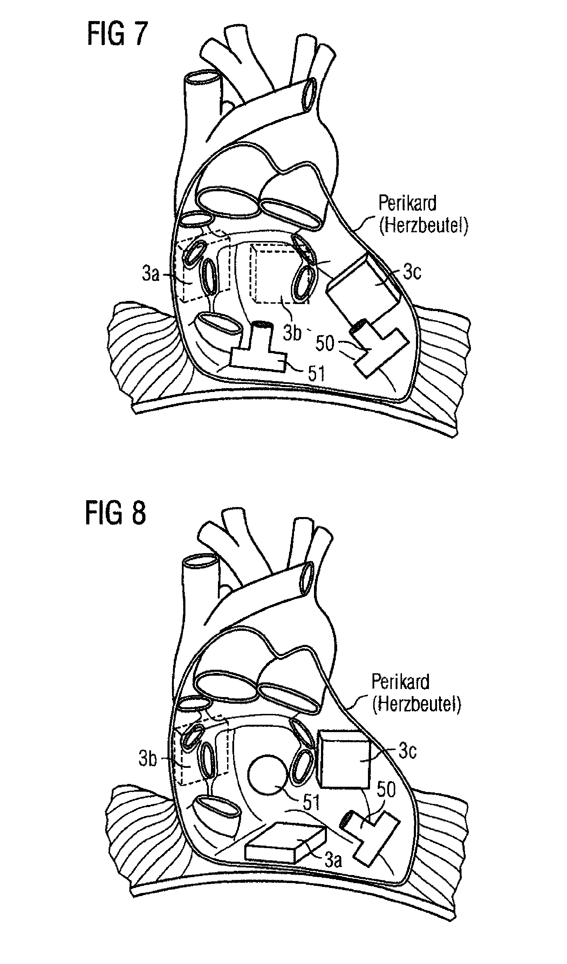

Die Figuren 7 bis 12 zeigen jeweils vorteilhafte Möglichkeiten zur Verwendung eines Kabelverbinders in Zusammenwirkung mit jeweils zwei Herzunterstützungspumpen, die jeweils einen in das linke oder rechte Herzventrikel hineinragenden Stutzen aufweisen. Der Kabelverbinder ist vorteilhaft jeweils mit Zuleitungskabeln der beiden Pumpen verbunden, die mit dem Kabelverbinder mittels Steckverbindungen verbunden sind.

Der Kabelverbinder kann vorteilhaft unmittelbar am Herzen, beispielsweise im Kontakt mit dem Herzbeutel, vorteilhaft auch innerhalb des Herzbeutels, angeordnet sein. Dies ist besonders dann günstig, wenn das Herz ohnehin durch eine Dysfunktion verkleinert ist.

Weiter kann der Kabelverbinder vorteilhaft am Herzbeutel befestigt, beispielsweise an diesem festgenäht sein.

Der Kabelverbinder sollte dabei einen ausreichenden Abstand zu den Herzunterstützungspumpen aufweisen.

Im Folgenden sind verschiedene Möglichkeiten für eine optimierte Platzierung des Kabelverbinders in Abhängigkeit von der Position der Herzunterstützungspumpen aufgezeigt, entsprechend den Darstellungen der Figuren 7 bis 10:
Figur 7: Platzierung Kabelverbinder und BVAD (Herzunterstützungspumpen für beide Ventrikel): Szenario 1
   1. LVAD (left ventricular assist device): Einlass-Stutzen der Pumpe in Apex des linken Ventrikels (LV)
   2. RVAD (right ventricular assist device): Einlass-Stutzen in inferiorer (diaphragmaler) Wand des rechten Ventrikels (RV)
   3. Kabelverbinder wird positioniert:
      a) Zwischen rechtem Vorhof (RA) und Perikard (Herzbeutel)
      b) Zwischen anteriorer (freier) Wand des RV und Brustbein (Sternum)
      c) Zwischen lateraler Wand des LV und Perikard.
Figur 8: Platzierung Kabelverbinder und BVAD: Szenario 2
   1. LVAD: Einlass-Stutzen der Pumpe 50 in Apex des linken Ventrikels (LV)
   2. RVAD: Einlass-Stutzen der Pumpe 51 in anteriorer (freier) Wand des rechten Ventrikels (RV)
   3. Kabelverbinder wird positioniert:
      a) Zwischen inferiorer (diaphragmaler) Wand des rechten Ventrikels (RV) und Pars diaphragmatica des Perikards
      b) Zwischen rechtem Vorhof (RA) und Perikard
      c) Zwischen lateraler Wand des LV und Perikard.

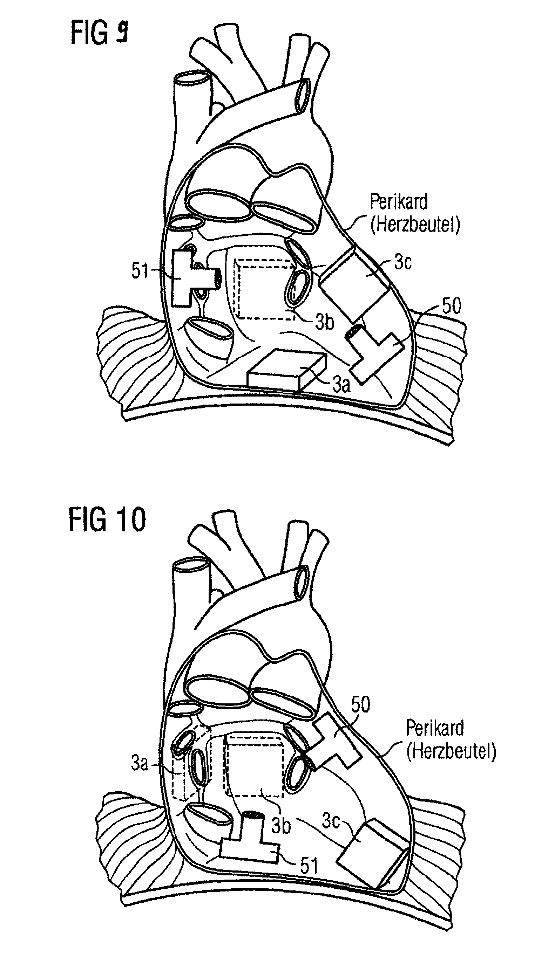

Figur 9: Platzierung Kabelverbinder und BVAD: Szenario 3
   1. LVAD: Einlass-Stutzen der Pumpe in Apex des linken Ventrikels (LV)
   2. RVAD: Einlass-Stutzen in rechtem Vorhof (RA)
   3. Kabelverbinder wird positioniert:
      a) Zwischen inferiorer (diaphragmaler) Wand des rechten Ventrikels (RV) und Pars diaphragmatica des Perikards
      b) Zwischen anteriorer (freier) Wand des RV und Brustbein (Sternum)
      c) Zwischen lateraler Wand des LV und Perikard.
Figur 10: Platzierung Kabelverbinder und BVAD: Szenario 4
   1. LVAD: Einlass-Stutzen der Pumpe in linkem Vorhof (LA)
   2. RVAD: Einlass-Stutzen in inferiorer (diaphragmaler) Wand des rechten Ventrikels (RV)
   3. Kabelverbinder wird positioniert:
      a) Zwischen rechtem Vorhof (RA) und Perikard
      b) Zwischen anteriorer (freier) Wand des RV und Brustbein (Sternum)
      c) Zwischen Apex des linken Ventrikels (LV) und Perikard.

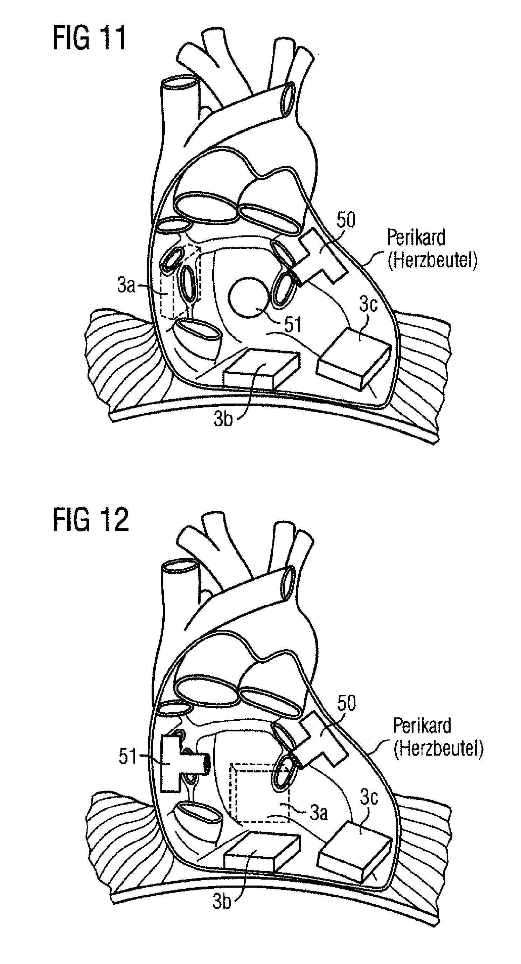

Figur 11: Platzierung Kabelverbinder und BVAD: Szenario 5
   1. LVAD: Einlass-Stutzen der Pumpe in linkem Vorhof (LA)
   2. RVAD: Einlass-Stutzen in anteriorer (freier) Wand des rechten Ventrikels (RV)
   3. Kabelverbinder wird positioniert:
      a) Zwischen rechtem Vorhof (RA) und Perikard
      b) Zwischen inferiorer (diaphragmaler) Wand des RV und Pars diaphragmatica des Perikards
      c) Zwischen Apex des linken Ventrikels (LV) und Perikard.
Figur 12: Platzierung Kabelverbinder und BVAD: Szenario 6
   1. LVAD: Einlass-Stutzen der Pumpe in linkem Vorhof (LA)
   2. RVAD: Einlass-Stutzen in rechtem Vorhof (RA)
   3. Kabelverbinder wird positioniert:
      a) Zwischen anteriorer (freier) Wand des rechten Ventrikels (RV) und Brustbein (Sternum)
      b) Zwischen inferiorer (diaphragmaler) Wand des RV und Pars diaphragmatica des Perikards
      c) Zwischen Apex des linken Ventrikels (LV) und Perikard.

## Patentansprüche

1. Implantierbare Kabelverbindungseinrichtung mit einem Kabelverbinder (2, 2', 2"), der zwei oder mehr Steckverbindungselemente (8, 8', 9, 9', 10, 10') aufweist, mit denen je ein implantierbares Kabel (3, 4, 6) durch eine Steckverbindung verbindbar ist, **dadurch gekennzeichnet, dass** der Kabelverbinders als steifer Körper aus einem biokompatiblen Material ausgebildet ist.

2. Kabelverbindungseinrichtung nach Anspruch 1, **dadurch gekennzeichnet, dass** mindestens zwei, insbesondere drei oder mehr Steckverbindungselemente (8, 8', 9, 9', 10, 10') auf derselben Seite des Kabelverbinders (2, 2', 2") angeordnet sind.

3. Kabelverbindungseinrichtung nach Anspruch 1 oder 2, **dadurch gekennzeichnet, dass** alle Steckverbindungselemente (8, 8', 9, 9', 10, 10') auf derselben Seite (21) des Kabelverbinders angeordnet sind.

4. Kabelverbindungseinrichtung nach Anspruch 1, 2 oder 3, **dadurch gekennzeichnet, dass** die Steckachsen (8a, 9a, 10a) der Steckverbindungselemente, die gemeinsam auf einer Seite (21) des Kabelverbinders angeordnet sind, parallel zueinander ausgerichtet sind.

5. Kabelverbindungseinrichtung nach einem der Ansprüche 1 bis 4, **dadurch gekennzeichnet, dass** der Kabelverbinders (2, 2', 2") eine als ebene Fläche ausgebildete Seite (21) aufweist, an der die Steckverbindungselemente angeordnet sind, und dass alle übrigen Begrenzungsflächen des Kabelverbinders konvex gerundet sind.

6. Kabelverbindungseinrichtung nach Anspruch 5, **dadurch gekennzeichnet, dass** der Kabelverbinder (2, 2', 2") im Wesentlichen halbkugelförmig abgerundet ausgebildet ist.

7. Kabelverbindungseinrichtung nach einem der Ansprüche 1 bis 6, **dadurch gekennzeichnet, dass** der Kabelverbinder (2, 2', 2") als Gehäuse (23) mit wenigstens einem Hohlraum ausgebildet ist.

8. Kabelverbindungseinrichtung nach Anspruch 7, **dadurch gekennzeichnet, dass** das Gehäuse (23) einen elektrischen Energiespeicher (27) und/oder ein elektrisch aktives Element (28, 29), insbesondere eine Einrichtung zur drahtlosen Kommunikation, aufnimmt.

9. Kabelverbindungseinrichtung nach einem der Ansprüche 1 bis 8, **dadurch gekennzeichnet, dass** der Kabelverbinder (2, 2', 2") an seinen Außenseiten ausschließlich aus Metall und/oder Glas und/oder Keramik besteht.

10. Kabelverbindungseinrichtung nach einem der Ansprüche 1 bis 9, **dadurch gekennzeichnet, dass** der Kabelverbinders (2, 2', 2") aus mehreren Teilen besteht, die zur Außenseite des Kabelverbinders gasdicht miteinander verschweißt und/oder vergossen sind.

11. Kabelverbindungseinrichtung nach einem der Ansprüche 1 bis 10, **gekennzeichnet durch** einen Kabelverbinder (2; 2', 2"), ein in diesen eingestecktes erstes perkutanes Kabel (3) und wenigstens ein in den Kabelverbinder eingestecktes zweites Kabel (4) sowie eine mit dem zweiten Kabel verbundene Blutpumpe (5) und insbesondere ein in den Kabelverbinder eingestecktes drittes Kabel (6) sowie eine mit dem dritten Kabel verbundene weitere Blutpumpe (7).

12. Verwendung einer Kabelverbindungseinrichtung nach einem der Ansprüche 1 bis 11, **dadurch gekennzeichnet, dass** die Kabelverbindungseinrichtung mit zwei Herzunterstützungspumpen verbunden und gemeinsam mit diesen unmittelbar an einem Herzen angeordnet, insbesondere an diesem oder am Herzbeutel fixiert ist.

13. Verfahren zur Implantierung einer Kabelverbindungseinrichtung nach einem der Ansprüche 1 bis 11, **dadurch gekennzeichnet, dass** der Kabelverbinder mit zwei Herzunterstützungspumpen elektrisch verbunden und zeitlich vorher oder danach am Herzbeutel eines Patientenherzens fixiert wird.
